# EUROPEAN PATENT SPECIFICATION

(11) **EP 2 024 008 B1**
(45) Date of publication and mention of the grant of the patent: **26.08.2015**
(21) Application number: 07710272.1
(22) Date of filing: 23.01.2007
(51) Int. Cl.: A61N 1/05, A61N 1/36

(54) **NOVEL MEDICAL ELECTRICAL LEAD FOR SPINAL CORD STIMULATION**
NEUE MEDIZINISCHE ELEKTROLEITUNG ZUR STIMULATION DES RÜCKENMARKS
NOUVELLE DÉRIVATION ÉLECTRIQUE MÉDICALE SERVANT À STIMULER LA MOELLE ÉPINIÈRE

(30) Priority: 28.04.2006 US 413581; 28.04.2006 US 413582
(43) Date of publication of application: 18.02.2009
(73) Proprietor: Medtronic, Inc., Minneapolis MN 55432-5604 (US)
(72) Inventor: METZLER, Michael, E., Minneapolis, MN 55408 (US); TOWER, Jessica, L., Minneapolis, MN 55406 (US); BOATWRIGHT, Mary, L., Andover, MN 55405 (US)
(74) Representative: Hughes, Andrea Michelle
(86) International application number: PCT/US2007/060904
(87) International publication number: WO 2007/127509

(56) References cited:
- US-A- 5 417 719
- US-A1- 2003 204 228
- US-A1- 2004 243 208
- US-A1- 2005 075 707
- US-A1- 2005 203 600

## Description

### TECHNICAL FIELD

The present invention is related to spinal cord stimulation therapy and more particularly to medical electrical leads for delivering the stimulation therapy.

### BACKGROUND

Electrical stimulation of a spinal cord, which induces pain-relieving paresthesia, can be provided by implantable systems that include electrodes coupled to elongate electrical leads. Such electrodes may be percutaneously or surgically introduced into the epidural space surrounding the spinal cord. Medical electrical leads which include an array of electrodes provide flexibility for selection from a variety of stimulation patterns upon implantation without having to physically reposition the lead within the epidural space. Such leads, wherein the electrode array is coupled along a body of the lead or along a distal paddle-like termination of the lead body, are known in the art. However, there is still a need for new spinal cord lead designs including distal ends that fit securely within the epidural space surrounding the spinal cord, and conform to the spinal cord so that an array or plurality of electrodes coupled to the distal end are in intimate contact with the dura mater enclosing the spinal cord.
US 2005/0203600 relates to stimulation electrode leads used for treating spinal disorders.
US 2005/0075707 relates to leads, tools and methods used with implantable stimulation systems.

### BRIEF DESCRIPTION OF THE DRAWINGS

The present invention provides a medical electrical lead as defined in claim 1.

### BRIEF DESCRIPTION OF THE DRAWINGS

The following drawings are illustrative of particular embodiments of the present invention and therefore do not limit the scope of the invention. The drawings are not to scale (unless so stated) and are intended for use in conjunction with the explanations in the following detailed description. Embodiments of the present invention will hereinafter be described in conjunction with the appended drawings, wherein like numerals denote like elements.
Figure 1A is a plan view of a medical electrical lead, according to some embodiments of the present invention.
Figure 1B is an end view of the lead shown in Figure 1A.
Figure 1C is a section view, through section line D-D of Figure 1A.
Figures 2A-B are a perspective views of the lead shown in Figure 1A.
Figure 3A is a schematic including a portion of the spinal canal cut away so that the lead of Figure 1A may be seen implanted in the epidural space surrounding a spinal cord.
Figure 3B is an end view of the implanted lead shown in Figure 3A.
Figure 4 is a plan view including a partial section of a portion of a lead body assembly, according to some embodiments of the present invention.
Figures 5A-B are plan views of mating mold halves, which may be used to couple lead bodies.

### DETAILED DESCRPTION

The following detailed description is exemplary in nature and is not intended to limit the scope, applicability, or configuration of the invention in any way. Rather, the following description provides practical illustrations for implementing exemplary embodiments of the present invention. Examples of constructions, materials, dimensions, and manufacturing processes are provided for selected elements, and all other elements employ that which is known to those of skill in the field of the invention. Those skilled in the art will recognize that many of the examples provided have suitable alternatives that can be utilized.

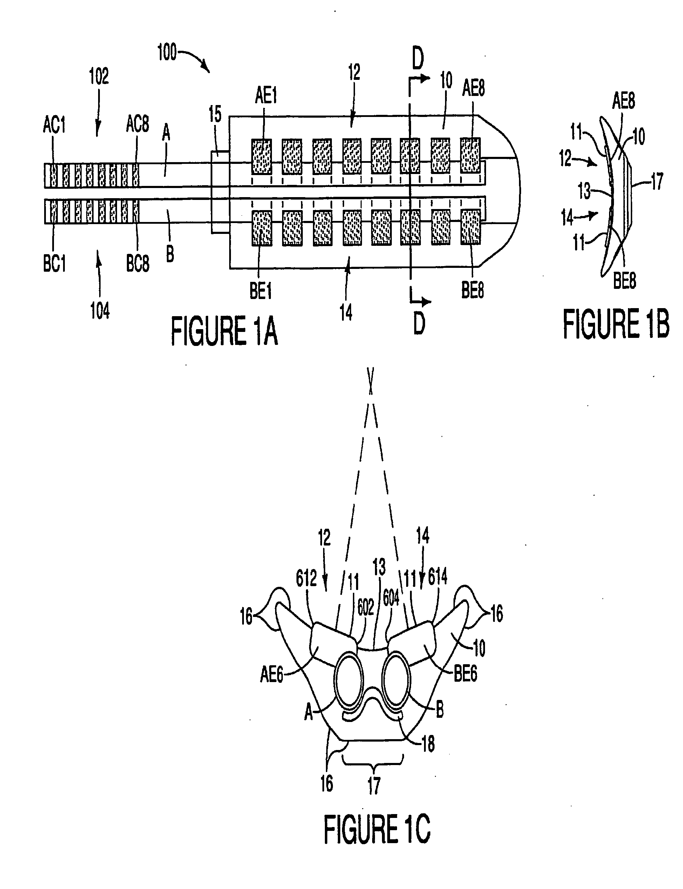

Figure 1A is a plan view of a medical electrical lead 100, according to some embodiments of the present invention. Figure 1A illustrates lead 100 including a first column 12 of electrodes AE1-AE8 and a second column 14 of electrodes BE1-BE8 coupled to an insulative distal portion 10, which may be formed from silicone rubber; a first lead body A and a second lead body B each extend proximally from distal portion 10 to corresponding connectors 102 and 104. According to the illustrated embodiment, each lead body A, B includes a plurality of insulated conductors; each conductor of each of the pluralities of conductors couples a corresponding electrode of electrodes AE1-AE8 and BE1-BE8 to a corresponding connector contact of contacts AC1-AC8 and BC1-BC8, according to methods known to those skilled in the art, which will be described in greater detail below. Those skilled in the art will appreciate that connectors 102 and 104 may either be connected directly to a stimulation device or to an extension, or adaptor, which includes one or two connectors for connection with the stimulation device. Figure 1A further illustrates a strain relief 15 extending proximally from distal portion 10 over lead bodies A, B.

Figure 1B is an end view of lead 100; and Figure 1C is a section view through section line D-D of Figure 1A. Figures 1B-C illustrate each of electrodes AE1-AE8 of first column 12 and each of electrodes BE1-BE8 of second column 14 including an approximately planar electrode surface 11; each electrode surface 11 of first column 12 is angled toward each electrode surface 11 of second column 14, such that planes (represented by dashed lines in Figure 1 C), which extend along and approximately orthogonally out from each column 12, 14 intersect one another. According to an exemplary embodiment of the present invention, a surface area of each electrode surface 11 is approximately six square millimeters, a longitudinal spacing between each electrode AE1-AE8 and BE1-BE8, within each column 12, 14 is approximately four millimeters, edge-to-edge, and approximately seven millimeters, center-to-center, and a spacing between columns 12 and 14 is between approximately three and four millimeters, center-to-center, and between approximately one and two millimeters, edge-to-edge. Although Figure 1A illustrates each electrode surface 11 of first column 12 longitudinally aligned with the corresponding electrode surface 11 of second column 14, the scope of the present invention is not so limited, and alternate embodiments may include staggered columns of electrode surfaces.

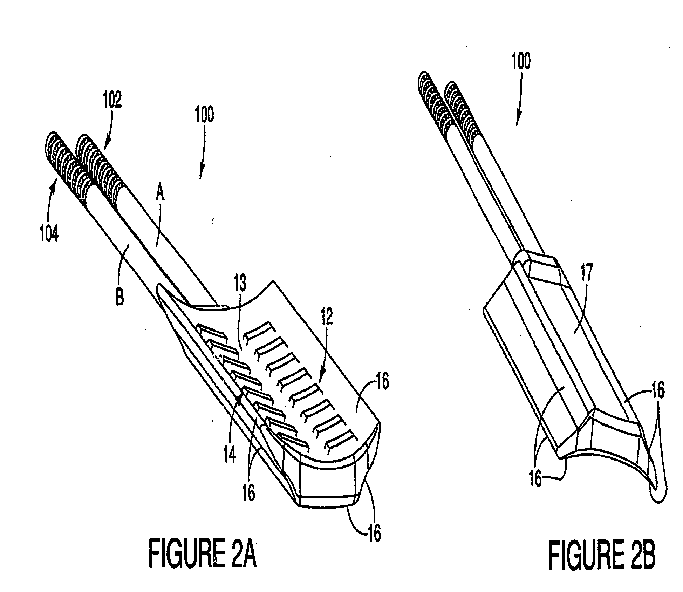

Figures 1B-C further illustrate distal portion 10 including a first surface 13 extending between an inner edge 602 of each electrode surface 11 of first column 12 and an inner edge 604 of each electrode surface 11 of second column 14, and a second surface 16 extending from an outer edge 612 of each electrode surface 11 of first column 12 to an outer edge 614 of each electrode surface 11 of second column 14. According to the illustrated embodiment, first surface 13 curves in a concave fashion and second surface 16 extends behind first and second columns 12, 14 and includes an approximately flat portion 17 disposed opposite first and second columns 12, 14. Figures 2A-B are perspective views of lead 100, which further illustrate first surface 13 and flat portion 17 of second surface 16. According to an exemplary embodiment, distal portion has a length between approximately sixty millimeters and approximately seventy millimeters, a width between approximately eight and approximately twelve millimeters, and a thickness, defined from flat portion 17 of surface 16 across to first surface 13, of approximately two millimeters; and, surface 13 has a radius of curvature of approximately nine millimeters.

Figure 1C further illustrates electrodes AE6 and BE6, representative of each electrode of columns 12 and 14, respectively, extending, in a cantilever fashion, laterally away from respective lead bodies A, B, which extend into distal portion 10. Each electrode AE1-AE8 and BE1-BE6 may be coupled, as such, for example, by laser welding, directly to the respective conductor within the corresponding lead body A, B, or to a conductive ring, which is coupled to the respective conductor and disposed about the corresponding lead body A, B. According to the illustrated embodiment, surface 11 of each electrode AE1-AE8 and BE1-BE6 protrudes slightly from adjacent portions of surfaces 13, 16, but, according to alternate embodiments, electrode surfaces 11 are flush with adjacent portions of surfaces 13, 16.

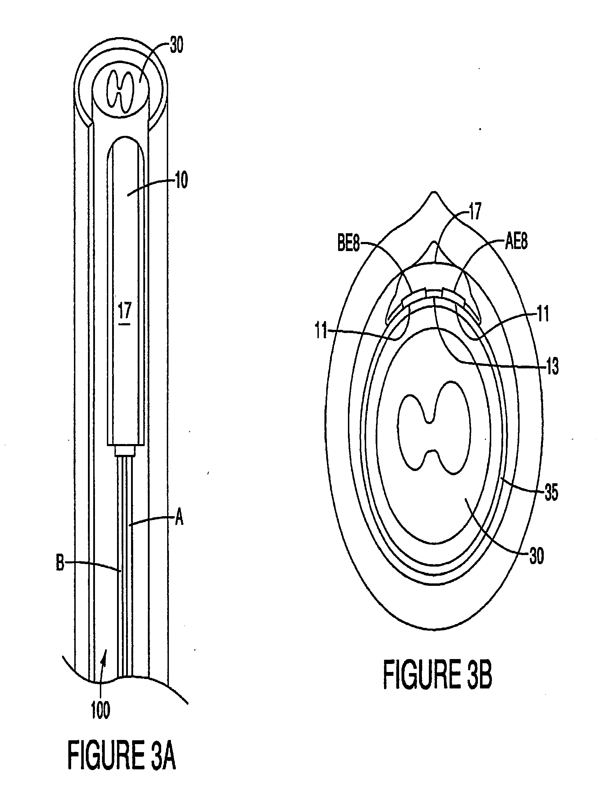

Figure 3A is a schematic including a portion of the spinal canal cut away so that lead 100 may be seen implanted in the epidural space of a spinal cord 30; and Figure 3B is an end view of the implanted lead 100. Figures 3A-B illustrate distal portion 10 of lead implanted in the epidural space such that electrode surfaces 11 are in intimate contact with the dura mater 35 surrounding spinal cord 30. Methods for implanting surgical spinal cord leads, such as lead 100, are well known to those skilled in the art. With reference to Figures 3A-B, it may be appreciated that the pre-formed profile of distal portion 10, along with the angled arrangement of electrode surfaces 11, as described in conjunction with Figure 1C, may greatly enhance the conformance of distal portion 10 about spinal cord 30 such that electrodes 11 contact the dura mater 35, as shown, for relatively efficient stimulation of spinal cord 30. Furthermore, the preformed shape of distal portion 10 facilitates the fit of distal portion 10 within the epidural space.

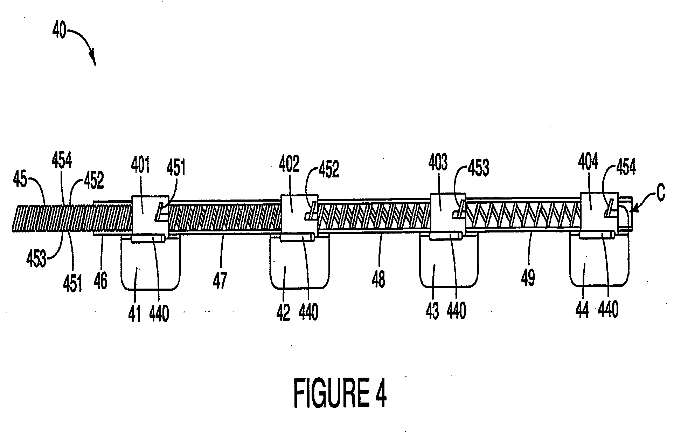

Figure 4 is a plan view including a partial section of a portion of a lead body assembly 40, according to some embodiments of the present invention. Figure 4 illustrates assembly 40 including four conductors 451-454 wound in a coil 45 and each conductor 451-454 coupled to a conductive ring 401-404, respectively, which is, in turn, coupled to an electrode 41-44, respectively. Conductors 451-454 are isolated from one another by an overlay of insulation material surrounding each of conductors 451-454, examples of which include, but are not limited to, polyimide, polytetrafluoroethylene (PTFE), and ethylene tetrafluoroethylene (ETFE). According to the illustrated embodiment, a distal end of each conductor 451-454 is hooked in a slot of the corresponding conductive ring 401-404, and laser welded thereto, and each electrode 41-44, whose active electrode surface is shown facing into the page, is coupled to the corresponding ring 401-404 via a laser weld, for example, as illustrated along line 440. Those skilled in the art will appreciate that conductor 45 extends proximally to a connector, for example, similar to connectors 102 and 104 of Figure 1A, where each conductor 451-454 is coupled to a corresponding connector contact, for example, in manner similar to that described for rings 401-404. Although Figure 4 illustrates assembly 40 including only four conductors and electrodes, it should be understood that lead body A or lead body B, of Figure 1A, which each include eight conductors and electrodes, may incorporate an assembly constructed in a similar manner to that of assembly 40, but including four additional conductors, rings and electrodes.

According to certain methods which do not form part of the present invention, a step of coupling electrodes to rings, for example, as illustrated in Figure 4, is performed prior to coupling two of lead body assemblies 40 together. According to some embodiments of the present invention, conductive rings 401-404 may form electrode surfaces of completed lead assemblies that include assembly 40, and have been originally manufactured for independent use, for example, as percutaneous spinal cord stimulation leads.

Figure 4 further illustrates insulation members 46, 47, 48 and 49 each extending around a portion of coil 45. According to some methods of the present invention, after electrodes 41-44 are coupled to rings 401-404, an adhesive, for example, silicone medical adhesive, is injected, per arrow C, to fill lumens of members 46, 47, 48 and 49, prior to coupling a two of assemblies 40 together.

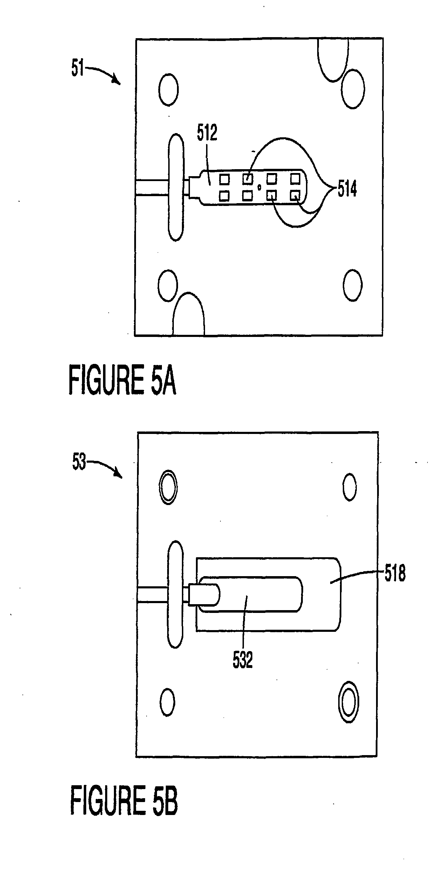

Coupling two of assemblies 40 together may be accomplished by forming a paddle-like insulative member around the pair assemblies 40 either by a transfer or an insert molding method, known to those skilled in the art; the paddle-like member, according to some embodiments is similar to distal portion 10 described in conjunction with Figures 1A-2B. Figures 5A-B are plan views of mating mold halves 51, 53, which may be used to couple lead bodies, via a transfer molding method, after electrodes 41-44 have been coupled to conductors 451-454. Figure 5A illustrates first mold half 51 including a cavity 512 for forming, in conjunction with a cavity 532 of mating mold half 53 shown in Figure 5B, a paddle-like insulative distal portion, for example, distal portion 10 illustrated in Figures 1A-2B.

Figure 5A further illustrates electrode reliefs 514 disposed at locations in cavity 512 to correspond with positions of electrode surfaces of electrodes 41-44 of each of two assemblies 40 that will be placed between mold halves 51, 53. Reliefs 514 prevent the flow of injected material, for example, MDX silicone rubber, which forms the paddle-like member, into sites corresponding to electrode surfaces of electrodes 41-44, so that the electrode surfaces will not be covered with the insulative material.

Figure 5B further illustrates mold half 53 including a relief 518 for placement of a sheet of mesh material, which will be embedded within the paddle-like member, for example, as illustrated in Figure 1C where the mesh is denoted by item number 18. Mesh 18 may enhance the structural integrity of the coupling of lead bodies A and B by distal portion 10. After the molding process is completed, excess mesh material may be trimmed away about the perimeter of the paddle-like member.

Referring back to Figures 1A-2B, it may be appreciated that a profile of cavity 512, in between reliefs 514, may have a convex curvature, for example, to form surface 13, and a profile of cavity 513 may include a flat portion, for example, to form surface 17. It should be noted that, although a transfer molding process calls for the injection of the material into mating cavities 512, 513 prior to insertion of assemblies 40 between mold halves 51, 53, other types of molding processes, which are within the scope of the present invention, for example, insert molding, call for insertion of assemblies 40 into a mold, prior to the injection of the material.

In the foregoing detailed description, the invention has been described with reference to specific embodiments. However, it may be appreciated that various modifications and changes can be made without departing from the scope of the invention as set forth in the appended claims.

## Claims

1. A medical electrical lead for spinal cord stimulation, comprising:
a first lead body (A) that includes a first plurality of conductors (451-454) isolated from one another and a first column (12) of electrodes (AE1-AE8, 41-44), each electrode of the first column being coupled to a corresponding conductor of the first plurality of conductors and including an electrode surface (11);
a second lead body (B) that includes a second plurality of conductors (451-454) isolated from one another and a second column (14) of electrodes (BE1-BE8, 41-44), each electrode of the second column being coupled to a corresponding conductor of the second plurality of conductors and including an electrode surface (11); and
an insulative distal portion (10) in which the first and second lead bodies extend, and to which the first and second columns of electrodes are coupled, the insulative distal portion including a first surface (13) extending between an inner edge (602) of each electrode surface of the first column and an inner edge (604) of each electrode surface of the second column;
**characterized in that**:
each electrode of the first column extends in a cantilever fashion, laterally away from the first lead body, and each electrode surface thereof is planar and extends from the inner edge thereof, laterally away from the first lead body, to an outer edge (612) thereof;
each electrode of the second column extends in a cantilever fashion, laterally away from the second lead body, and each electrode surface thereof is planar and extends from the inner edge thereof, laterally away from the second lead body, to an outer edge (614) thereof; and
each planar electrode surface of the first and second columns is exposed along a first side of the distal portion, and each planar electrode surface of the first column is angled toward each planar electrode surface of the second column such that a plane extending along the first column and orthogonally out from the electrode surfaces of the first column intersects a plane extending along the second column and orthogonally out from the electrode surfaces of the second column.

2. The lead of claim 1, wherein the first surface of the distal portion is concave; and further **characterized in that** a second surface (1b) of the insulative distal portion includes a flat portion 817) opposite the concave first surface.

3. The lead of claim 1 or 2, further comprising:
a first connector (102) extending proximally from the first lead body and including a first plurality of contact surfaces (AC1-AC8), each contact surface coupled to a corresponding electrode of the first column via a corresponding conductor of the first plurality of conductors; and
a second connector (104) extending proximally from the second lead body and including a second plurality of contact surfaces (BC1-BC8), each contact surface coupled to a corresponding electrode of the second column via a corresponding conductor of the second plurality of conductors.

4. The lead of claim 1, 2 or 3, wherein the first lead body further includes a first plurality of conductive rings (401-404) longitudinally spaced apart from one another and separated from one another by a first set of insulating spacers (47-49), each ring of the first plurality of conductive rings being coupled to a corresponding conductor of the first plurality of conductors; and the second lead body further includes a second plurality of conductive rings (401-404) longitudinally spaced apart from one another and separated from one another by a second set of insulating spacers (47-49), each ring of the second plurality of conductive rings being coupled to a corresponding conductor of the second plurality of conductors; and further **characterized in that**:
each electrode of the first column is coupled to a corresponding ring of the first plurality of rings; and
each electrode of the second column is coupled to a corresponding ring of the second plurality of rings.

5. The lead of claim 1 or 2, further comprising a mesh panel (18) extending between the first and second lead bodies within the distal portion.

## Patentansprüche

1. Medizinische elektrische Leitung zur Rückenmarkstimulation, die Folgendes enthält:
einen ersten Leitungskörper (A), der mehrere erste von einander isolierte Leiter (451-454) und eine erste Spalte (12) von Elektroden (AE1-AE8, 41-44) enthält, wobei jede Elektrode der ersten Spalte an einen entsprechenden Leiter der mehreren ersten Leiter gekoppelt ist und eine Elektrodenoberfläche (11) aufweist;
einen zweiten Leitungskörper (B), der mehrere zweite von einander isolierte Leiter (451-454) und eine zweite Spalte (14) von Elektroden (BE1-BE8, 41-44) enthält, wobei jede Elektrode der zweiten Spalte an einen entsprechenden Leiter der mehreren zweiten Leiter gekoppelt ist und eine Elektrodenoberfläche (11) aufweist; und
einen isolierenden distalen Abschnitt (10), in dem sich der erste und der zweite Körper erstrecken, und an den die erste und zweite Spalte der Elektroden gekoppelt sind, wobei der isolierende distale Abschnitt eine erste Oberfläche (13) aufweist, die sich zwischen einer Innenkante (602) jeder Elektrodenoberfläche der ersten Spalte und einer Innenkante (604) jeder Elektrodenoberfläche der zweiten Spalte erstreckt;
**dadurch gekennzeichnet, dass**:
jede Elektrode der ersten Spalte sich freitragend, seitlich von dem ersten Leitungskörper weg erstreckt und jede Elektrodenoberfläche hiervon planar ist und sich von der Innenkante hiervon, seitlich von dem ersten Leitungskörper weg zu einer Außenkante (612) hiervon erstreckt;
jede Elektrode der zweiten Spalte sich freitragend, seitlich von dem zweiten Leitungskörper weg erstreckt und jede Elektrodenoberfläche hiervon planar ist und sich von der Innenkante hiervon, seitlich von dem zweiten Leitungskörper weg zu einer Außenkante (614) hiervon erstreckt; und
jede planare Elektrodenoberfläche der ersten und der zweiten Spalte entlang einer ersten Seite des distalen Abschnitts freiliegen und jede planare Elektrodenoberfläche der ersten Spalte winkelförmig auf jede planare Elektrodenoberfläche der zweiten Spalte ausgerichtet ist, so dass eine Ebene, die sich entlang der ersten Spalte und senkrecht von den Elektrodenoberflächen der ersten Spalte erstreckt, eine Ebene schneidet, die sich entlang der zweiten Spalte und senkrecht von den Elektrodenoberflächen der zweiten Spalte erstreckt.

2. Leitung nach Anspruch 1, wobei die erste Oberfläche des distalen Abschnitts konkav ist; und ferner **dadurch gekennzeichnet, dass** eine zweite Oberfläche (1b) des isolierenden distalen Abschnitts einen flachen Abschnitt (17), der der konkaven ersten Oberfläche gegenüberliegt, enthält.

3. Leitung nach Anspruch 1 oder 2, die ferner Folgendes enthält:
ein erstes Verbindungsstück (102), das sich von dem ersten Leitungskörper proximal erstreckt und mehrere erste Kontaktoberflächen (AC1-AC8) enthält, wobei jede Kontaktoberfläche über einen entsprechenden Leiter der mehreren ersten Leiter an eine entsprechende Elektrode der ersten Spalte gekoppelt ist; und
ein zweites Verbindungsstück (104), das sich von dem zweiten Leitungskörper proximal erstreckt und mehrere zweite Kontaktoberflächen (BC1-BC8) enthält, wobei jede Kontaktoberfläche über einen entsprechenden Leiter der mehreren zweiten Leiter an eine entsprechende Elektrode der zweiten Spalte gekoppelt ist.

4. Leitung nach Anspruch 1, 2 oder 3, wobei der erste Leitungskörper ferner mehrere erste leitfähige Ringe (401-404) enthält, die in Längsrichtung voneinander entfernt sind und durch eine erste Menge von isolierenden Abstandshaltern (47-49) voneinander getrennt sind, wobei jeder Ring der mehreren ersten leitfähigen Ringe an eine entsprechende Leiter der mehreren ersten Leiter gekoppelt ist; und der zweite Leitungskörper ferner mehrere zweite leitfähige Ringe (401-404) enthält, die in Längsrichtung voneinander entfernt sind und durch eine zweite Menge von isolierenden Abstandshaltern (47-49) voneinander getrennt sind, wobei jeder Ring der mehreren zweiten leitfähigen Ringe an eine entsprechende Leiter der mehreren zweiten Leiter gekoppelt ist; und ferner **dadurch gekennzeichnet, dass**:
jede Elektrode der ersten Spalte an einen entsprechenden Ring der mehreren ersten Ringe gekoppelt ist; und
jede Elektrode der zweiten Spalte an einen entsprechenden Ring der mehreren zweiten Ringe gekoppelt ist.

5. Leitung nach Anspruch 1 oder 2, die ferner eine Gitterplatte (18) enthält, die sich innerhalb des distalen Abschnitts zwischen dem ersten und dem zweiten Leitungskörper erstreckt.

## Revendications

1. Fil électrique médical pour une stimulation de moelle épinière, comportant :
un premier corps de fil (A) qui inclut une première pluralité de conducteurs (451 à 454) isolés les uns des autres et une première colonne (12) d'électrodes (AE1 à AE8, 41 à 44), chaque électrode de la première colonne étant couplée à un conducteur correspondant de la première pluralité de conducteurs et incluant une surface d'électrode (11) ;
un second corps de fil (B) qui inclut une seconde pluralité de conducteurs (451 à 454) isolés les uns des autres et une seconde colonne (14) d'électrodes (BE1 à BE8, 41 à 44), chaque électrode de la seconde colonne étant couplée à un conducteur correspondant de la seconde pluralité de conducteurs et incluant une surface d'électrode (11) ; et
une portion distale isolante (10) dans laquelle les premier et second corps de fil s'étendent, et à laquelle les première et seconde colonnes d'électrode sont couplées, la portion distale isolante incluant une première surface (13) s'étendant entre un bord intérieur (602) de chaque surface d'électrode de la première colonne et un bord intérieur (604) de chaque surface d'électrode de la seconde colonne ;
**caractérisé en ce que** :
chaque électrode de la première colonne s'étend en porte-à-faux, latéralement en s'écartant du premier corps de fil, et chaque surface d'électrode de celle-ci est plane et s'étend depuis le bord intérieur de celle-ci, latéralement en s'écartant du premier corps de fil, jusqu'à un bord extérieur (612) de celle-ci ;
chaque électrode de la seconde colonne s'étend en porte-à-faux, latéralement en s'écartant du second corps de fil, et
chaque surface d'électrode de celle-ci est plane et s'étend depuis le bord intérieur de celle-ci, latéralement en s'écartant du second corps de fil, jusqu'à un bord extérieur (614) de celle-ci ; et
chaque surface d'électrode plane des première et seconde colonnes est exposée le long d'un premier côté de la portion distale, et chaque surface d'électrode plane de la première colonne est inclinée vers chaque surface d'électrode plane de la seconde colonne de telle sorte qu'un plan s'étendant le long de la première colonne et orthogonalement en dehors des surfaces d'électrode de la première colonne coupe un plan s'étendant le long de la seconde colonne et orthogonalement en dehors des surfaces d'électrode de la seconde colonne.

2. Fil selon la revendication 1, dans lequel la première surface de la portion distale est concave, et en outre **caractérisé en ce qu'**une seconde surface (1b) de la portion distale isolante inclut une portion plate (817) opposée à la première surface concave.

3. Fil selon la revendication 1 ou 2, comportant en outre :
un premier connecteur (102) s'étendant proximalement à partir du premier corps de fil et incluant une première pluralité de surfaces de contact (AC1 à AC8), chaque surface de contact étant couplée à une électrode correspondante de la première colonne via un conducteur correspondant de la première pluralité de conducteurs ; et
un second connecteur (104) s'étendant proximalement à partir du second corps de fil et incluant une seconde pluralité de surfaces de contact (BC1 à BC8), chaque surface de contact étant couplée à une électrode correspondante de la seconde colonne via un conducteur correspondant de la seconde pluralité de conducteurs.

4. Fil selon la revendication 1, 2 ou 3, dans lequel le premier corps de fil inclut en outre une première pluralité de bagues conductrices (401 à 404) longitudinalement espacées les unes des autres et séparées les unes des autres par un premier ensemble d'organes d'espacement isolants (47 à 49), chaque bague de la première pluralité de bagues conductrices étant couplée à un conducteur correspondant de la première pluralité de conducteurs ; et le second corps de fil inclut en outre une seconde pluralité de bagues conductrices (401 à 404) longitudinalement espacées les unes des autres et séparées les unes des autres par un second ensemble d'organes d'espacement isolants (47 à 49), chaque bague de la seconde pluralité de bagues conductrices étant couplée à un conducteur correspondant de la seconde pluralité de conducteurs ; **caractérisé en ce que** :
chaque électrode de la première colonne est couplée à une bague correspondante de la première pluralité de bagues ; et
chaque électrode de la seconde colonne est couplée à une bague correspondante de la seconde pluralité de bagues.

5. Fil selon la revendication 1 ou 2, comportant en outre un panneau à mailles (18) s'étendant entre les premier et second corps de fil à l'intérieur de la portion distale.
